# EUROPEAN PATENT APPLICATION

(11) **EP 2 965 757 A1**
(43) Date of publication of application: **13.01.2016**
(21) Application number: 15178064.0
(22) Date of filing: 03.02.2012
(51) Int. Cl.: A61K 31/337, A61K 31/4188, A61K 31/506, A61K 45/06, A61P 35/00, A61P 43/00

(54) **Pharmaceutical compositions for treating malignant glioma**

(30) Priority: 04.02.2011 WO PCT/IB2011/050494
(62) Divisional of application: 12706925.0
(71) Applicant: Actelion Pharmaceuticals Ltd., 4123 Allschwil (CH)
(72) Inventor: Regenass, Urs, 4123 Allschwil (CH)
(74) Representative: Ruhlmann, Eric

(57) **Abstract**

The invention relates to the use of macitentan in combination with radiotherapy or with both radiotherapy and a cytotoxic therapy agent (such as temozolomide or paclitaxel) in the treatment of malignant glioma, in particular glioblastoma multiforme.

## Description

The present invention concerns the combination of macitentan, i.e. the compound of formula I 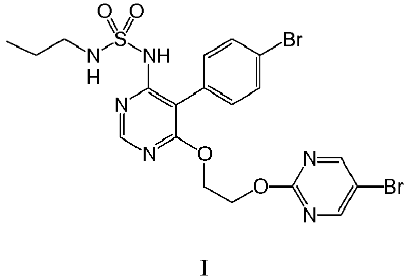 with a chemotherapeutic agent such as temozolomide or paclitaxel, with radiotherapy or with both a chemotherapeutic agent and radiotherapy, for therapeutic use, simultaneously, separately or over a period of time, in the treatment of malignant glioma, in particular glioblastoma multiforme.

Malignant glioma and glioblastoma multiforme constitute rare cancer forms for which there are currently no satisfactory treatments. Even with the best available therapy, the prognosis for the patients remains poor (see e.g. M. Khasraw and A.B. Lassman, Curr. Oncol. Rep. (2010), 12, 26-33).

PCT publication WO 02/053557 describes endothelin receptor antagonists including the compound of formula (I) (the International Nonproprietary Name of which is macitentan) and the use of said endothelin receptor antagonists in the treatment of various diseases, including cancer in general.

Besides, PCT publication WO 2009/104149 discloses that the combination of macitentan and paclitaxel has a synergistic effect in the treatment of ovarian cancer.

Temozolomide (the active principle of a medicament sold under the trademarks Temodar® in the United States) is an oral alkylating agent. This compound is currently approved in the European Union and the United States for, among others, the treatment of glioblastoma multiforme.

The potential role of endothelins, in particular endothelin-1 (ET-1), in malignant glioma and in glioblastoma multiforme is known from literature and endothelin receptor antagonists in general have been mentioned as potential therapeutic agents for treating glioblastoma multiforme.

For example, G. Egidy et al (Lab. Invest. (2000), 80, 1681-1689) teach that "[in] human glioblastoma cell lines, the ET-1 system was not involved in tumor proliferation, but ET-1 was a survival factor (...)".

Besides, M. Paolillo et al (Pharmacol. Res. (2010), 61, 306-315) teach that "in glioma cell lines ETB antagonists reduce proliferation and induce apoptosis". However the authors then admit that "(f)uture studies (...) are required to confirm the reliability of ETB antagonists as a promising pharmacological tool in glioma treatment".

On the other hand, the endothelin receptor antagonist atrasentan has been studied in patients having recurrent malignant glioma in phase I clinical studies, as reported by Phuphanich et al (J Neurooncol. (2008). 10(4), 617-623). However the development of this compound in this indication has not been continued to date.

The applicant has now found that macitentan, which is both an endothelin receptor A (ET_{A}R) and an endothelin receptor B (ET_{B}R) antagonist, produces exceptionally high effects in an *in vivo* model of glioblastoma when combined with a cytotoxic chemotherapy agent such as temozolomide or paclitaxel. As a result, macitentan in combination with a cytotoxic chemotherapy agent such as temozolomide or paclitaxel may be used for the preparation of a medicament, and is suitable, for the treatment of malignant glioma, in particular glioblastoma multiforme.

Various embodiments of the invention are presented hereafter:
1) The invention firstly relates to a product containing macitentan, or a pharmaceutically acceptable salt of this compound, for use in the treatment of malignant glioma in combination with a cytotoxic chemotherapy agent, with radiotherapy or with both a cytotoxic therapy agent and radiotherapy. According to this invention, the components of the combination may be administered simultaneously, separately or over a period of time.

The following paragraphs provide definitions of the various terms used in the present patent application and are intended to apply uniformly throughout the specification and claims, unless an otherwise expressly set out definition provides a broader or narrower definition.

The term "pharmaceutically acceptable salt" refers to non-toxic, inorganic or organic acid and/or base addition salts. Reference can be made to "Salt selection for basic drugs", Int. J. Pharm. (1986), 33, 201-217.

The term "cytotoxic chemotherapy agent" as used herein refers to a substance inducing apoptosis or necrotic cell death. Examples of cytotoxic chemotherapy agents which may be used in combination with ERAs in accordance to the present invention include:
▪ alkylating agents (for example mechlorethamine, chlorambucil, cyclophosphamide, ifosfamide, streptozocin, carmustine, lomustine, melphalan, busulfan, dacarbazine, temozolomide, thiotepa or altretamine);
▪ platinum drugs (for example cisplatin, carboplatin or oxaliplatin);
▪ antimetabolite drugs (for example 5-fluorouracil, capecitabine, 6-mercaptopurine, methotrexate, gemcitabine, cytarabine, fludarabine or pemetrexed);
▪ anti-tumor antibiotics (for example daunorubicin, doxorubicin, epirubicin, idarubicin, actinomycin-D, bleomycin, mitomycin-C or mitoxantrone); and
▪ mitotic inhibitors (for example paclitaxel, docetaxel, ixabepilone, vinblastine, vincristine, vinorelbine, vindesine or estramustine); and
▪ topoisomerase inhibitors (for example etoposide, teniposide, topotecan, irinotecan, diflomotecan or elomotecan).

"Simultaneously" or "simultaneous", when referring to an administration type, means in the present application that the administration type concerned consists in the administration of two or more active ingredients by the same route and at the same time.

"Separately" or "separate", when referring to an administration type, means in the present application that the administration type concerned consists in the administration of two or more active ingredients at approximately the same time by at least two different routes.

By administration "over a period of time" is meant in the present application the administration of two or more active ingredients at different times, and in particular an administration method according to which the entire administration of one of the active ingredients is completed before the administration of the other or others begins. In this way it is possible to administer one of the active ingredients for several months before administering the other active ingredient or ingredients. In this case, no simultaneous administration occurs. Administration "over a period of time" also encompasses situations wherein the active ingredients are not given with the same periodicity (e.g. wherein one active ingredient is given once a day and another is given once a week).
2) In particular, the malignant glioma of embodiment 1) will be glioblastoma multiforme.
3) Preferably, the product of embodiment 1) or 2) will be combined with a cytotoxic therapy agent or both a cytotoxic therapy agent and radiotherapy, said cytotoxic therapy agent being selected from alkylating agents and mitotic inhibitors.
4) According to one variant of embodiment 3), the cytotoxic therapy agent will be an alkylating agent (notably an alkylating agent selected from mechlorethamine, chlorambucil, cyclophosphamide, ifosfamide, streptozocin, carmustine, lomustine, melphalan, busulfan, dacarbazine, temozolomide, thiotepa, altretamine and pharmaceutically acceptable salts of these compounds).
5) In particular, the alkylating agent of embodiment 4) will be temozolomide or a pharmaceutically acceptable salt thereof.
6) According to another variant of embodiment 3), the cytotoxic therapy agent will be a mitotic inhibitor (and notably a mitotic inhibitor selected from paclitaxel, docetaxel, ixabepilone, vinblastine, vincristine, vinorelbine, vindesine, estramustine and pharmaceutically acceptable salts of these compounds).
7) In particular, the mitotic inhibitor of embodiment 6) will be paclitaxel or a pharmaceutically acceptable salt thereof.
8) According to one variant of embodiments 1) to 7), macitentan or its pharmaceutically acceptable salt will be intended to be administered by oral route.
9) According to another variant of embodiments 1) to 7), macitentan or its pharmaceutically acceptable salt will be intended to be administered by intravenous or intraperitoneal route (and notably by intravenous route).
10) In one particular sub-embodiment, the product according to one of embodiments 1) to 9) will be such that the cytotoxic therapy agent is intended to be administered by oral route.
11) In another particular sub-embodiment, the product according to one of embodiments 1) to 4) will be such that the cytotoxic therapy agent is intended to be administered by intravenous or intraperitoneal route (and notably by intravenous route).
12) The invention also relates to a pharmaceutical composition containing, as active principles, macitentan, or a pharmaceutically acceptable salt of this compound, in combination with a cytotoxic therapy agent, as well as at least one non-toxic excipient, for use, optionally in combination with radiotherapy, in the treatment of malignant glioma.
13) In particular, the pharmaceutical composition of embodiment 12) will be for use in the treatment of glioblastoma multiforme.
14) Preferably, the cytotoxic therapy agent of embodiment 12) or 13) will be selected from alkylating agents and mitotic inhibitors.
15) According to one variant of embodiment 14), the cytotoxic therapy agent will be an alkylating agent (notably an alkylating agent selected from mechlorethamine, chlorambucil, cyclophosphamide, ifosfamide, streptozocin, carmustine, lomustine, melphalan, busulfan, dacarbazine, temozolomide, thiotepa, altretamine and pharmaceutically acceptable salts of these compounds).
16) In particular, the alkylating agent of embodiment 15) will be temozolomide or a pharmaceutically acceptable salt thereof.
17) According to another variant of embodiment 14), the cytotoxic therapy agent will be a mitotic inhibitor (and notably a mitotic inhibitor selected from paclitaxel, docetaxel, ixabepilone, vinblastine, vincristine, vinorelbine, vindesine, estramustine and pharmaceutically acceptable salts of these compounds).
18) In particular, the mitotic inhibitor of embodiment 17) will be paclitaxel or a pharmaceutically acceptable salt thereof.
19) In one preferred sub-embodiment, the pharmaceutical composition according to one of embodiments 12) to 18) will be in a liquid form suitable for intravenous administration.
20) The pharmaceutical composition of embodiment 19) may for example contain macitentan or a pharmaceutically acceptable salt of this compound and temozolomide or a pharmaceutically acceptable salt thereof, in a water solution containing mannitol, L-threonine, polysorbate 80, sodium citrate and hydrochloric acid (such water solution being for example obtained by mixing commercial TEMODAR^{®} for injection with water for injection and adding macitentan or a pharmaceutically acceptable salt thereof).
21) The pharmaceutical composition of embodiment 19) may also contain macitentan or a pharmaceutically acceptable salt of this compound and temozolomide or a pharmaceutically acceptable salt thereof, in a water solution containing methylcellulose, polysorbate 80, glucose, polyoxyethylated castor oil (e.g. Cremophor® EL) and ethanol.
22) In another preferred sub-embodiment, the pharmaceutical composition according to one of embodiments 12) to 18) will be in a solid form suitable for oral administration.
23) In an alternative embodiment of this invention, macitentan may be formulated as a tablet as described in WO 2007/031933, whereas the cytotoxic therapy agent may be in one of its commercial formulations (preferably an oral formulation if available), the combination being intended for therapeutic use, simultaneously, separately or over a period of time, in the treatment of malignant glioma, in particular glioblastoma multiforme.

The production of the pharmaceutical compositions can be effected in a manner which will be familiar to any person skilled in the art (see for example Remington, The Science and Practice of Pharmacy, 21st Edition (2005), Part 5, "Pharmaceutical Manufacturing" [published by Lippincott Williams & Wilkins]) by bringing the described compounds or their pharmaceutically acceptable salts, optionally in combination with other therapeutically valuable substances, into a galenical administration form together with suitable, non-toxic, inert, therapeutically compatible solid or liquid carrier materials and, if desired, usual pharmaceutical adjuvants.
24) The invention further relates to the use of macitentan, or a pharmaceutically acceptable salt of this compound, in combination with a cytotoxic therapy agent, for the manufacture of a medicament intended to be used, optionally in combination with radiotherapy, in the treatment of malignant glioma.
25) In particular, the malignant glioma of embodiment 24) will be glioblastoma multiforme.
26) Preferably, the cytotoxic therapy agent of embodiment 24) or 25) will be selected from alkylating agents and mitotic inhibitors.
27) According to one variant of embodiment 26), the cytotoxic therapy agent will be an alkylating agent (notably an alkylating agent selected from mechlorethamine, chlorambucil, cyclophosphamide, ifosfamide, streptozocin, carmustine, lomustine, melphalan, busulfan, dacarbazine, temozolomide, thiotepa, altretamine and pharmaceutically acceptable salts of these compounds).
28) In particular, the alkylating agent of embodiment 27) will be temozolomide, or a pharmaceutically acceptable salt thereof.
29) According to another variant of embodiment 26), the cytotoxic therapy agent will be a mitotic inhibitor (and notably a mitotic inhibitor selected from paclitaxel, docetaxel, ixabepilone, vinblastine, vincristine, vinorelbine, vindesine, estramustine and pharmaceutically acceptable salts of these compounds).
30) In particular, the mitotic inhibitor of embodiment 29) will be paclitaxel or a pharmaceutically acceptable salt thereof.
31) The invention further relates to the use of macitentan, or a pharmaceutically acceptable salt of this compound, or a pharmaceutically acceptable salt thereof, for the manufacture of a medicament intended to be used in combination with radiotherapy with the treatment malignant glioma.
32) In particular, the malignant glioma of embodiment 31) will be glioblastoma multiforme.
33) The invention further relates to a method of treating a patient having malignant glioma by administering to said patient a combination of macitentan or a pharmaceutically acceptable salt of this compound, with a cytotoxic therapy agent, with radiotherapy, or with both a cytotoxic therapy agent and radiotherapy.
34) In particular, the malignant glioma of embodiment 33) will be glioblastoma multiforme.
35) Preferably, the cytotoxic therapy agent of embodiment 33) or 34) will be selected from alkylating agents and mitotic inhibitors.
36) According to one variant of embodiment 35), the cytotoxic therapy agent will be an alkylating agent (notably an alkylating agent selected from mechlorethamine, chlorambucil, cyclophosphamide, ifosfamide, streptozocin, carmustine, lomustine, melphalan, busulfan, dacarbazine, temozolomide, thiotepa, altretamine and pharmaceutically acceptable salts of these compounds).
37) In particular, the alkylating agent of embodiment 36) will be temozolomide, or a pharmaceutically acceptable salt thereof.
38) According to another variant of embodiment 35), the cytotoxic therapy agent will be a mitotic inhibitor (and notably a mitotic inhibitor selected from paclitaxel, docetaxel, ixabepilone, vinblastine, vincristine, vinorelbine, vindesine, estramustine and pharmaceutically acceptable salts of these compounds).
39) In particular, the mitotic inhibitor of embodiment 38) will be paclitaxel or a pharmaceutically acceptable salt thereof.

Besides, preferences indicated for the product according to this invention of course apply *mutatis mutandis* to the pharmaceutical compositions, uses and methods according to this invention.

Though the exact administration doses of the active principle(s) of a product according to this invention will have to be determined by the treating physician, it is expected that a dose of 0.01 to 10 mg (and preferably 0.1 to 5 mg and more preferably 0.1 to 1 mg) of macitentan per kg of patient body weight per day optionally combined with, for example, a dose of 1 to 20 mg (and preferably 2 to 15 mg) of temozolomide per kg of patient body weight per day, or a dose of 0.1 to 10 mg (and preferably 1 to 3 mg) of paclitaxel per kg of patient body weight per day, will be appropriate.

Examples showing the usefulness of the invention are described in the following section, which serves to illustrate the invention in more detail without limiting its scope in any way.

### Pharmacological properties of the invention product

### Examples 1 to 3: LN229 human glioblastoma cells stereotactically injected into nude mice brain:

### Materials

### Vehicle for macitentan:

The vehicle for macitentan consisted in 0.5% methylcellulose and 0.05% Tween 80. Tween 80 was added to the appropriate volume of sterile 0.5% methylcellulose solution before each preparation of macitentan. Macitentan was weighed and homogenised with vehicle for one hour at room temperature to obtain a fine and homogeneous suspension at 2 mg/ml. The working solutions of macitentan were prepared every week. Seven aliquots were prepared for each day of treatment, stored at 4°C and protected from light. After each use, remaining solution was not kept for another use and was destroyed.

### Vehicle for temozolomide:

Temozolomide (100 mg) was prepared first in Cremophor EL/Ethanol (2:1 w/w). After complete dilution, temozolomide was diluted in glucose 5% (Aguettant, France) to reach the concentration of 0.25 mg/ml. The working solutions of temozolomide was prepared prior to each administration to mice and not kept for another use.

### Administration routes

### Administration route for macitentan:

Macitentan was administered by oral gavage *via* a canula at 5 ml/kg. The administration volumes were adapted according to the most recent individual body weight of mice.

### Administration route for temozolomide:

Temozolomide was administered by oral gavage *via* a canula at 10 ml/kg. The administration volume was adapted according to the most recent individual body weight of mice.

### Animals

Forty-eight (48) female Swiss *Nude* mice were obtained from Charles River (France). Animals were observed for 7 days in a specific-pathogen-free animal care unit before treatment during the acclimatization period. Animal experiments were performed according to the European ethical guidelines of animal experimentation.

### General method used for stereotactic injections of human glioblastoma LN229 cells into nude mice brain

Cultured human glioblastoma LN229 cells were harvested by a brief exposure to 0.02%EDTA-0.25%Trypsin and re-suspended in Mg⁺⁺/Ca⁺⁺ free Hank's Balanced Salt Solution (adjusted to 1x10⁵ cells in 10µl). Nude mice were anesthetized by the intraperitoneal injection of Nembutal (0.5 mg/g body weight, Abbott Laboratories, North Chicago, IL) for stereotactic intrathecal injection of 1x10⁵ LN229 cells/mouse. In brief, a single incision was made from the anterior pole of the skull to the posterior ridge to expose the scalp. A hole was drilled over the target area and LN229 cells were injected using the following stereotactic coordinates, all relative to the bregma: 1.5 mm rostral, 1.5 mm anterioa and 4 mm below the pial surface. An automated micro-pump (Stoelting Instruments, Wood Dale, IL) was used to dispense 1x10⁵ cells in 10 µl suspension over a 5 minute period. After the injection, the hole was plugged with bone wax and skin was closed with skin staples.

### Example 1: treatment with macitentan and temozolomide:

Two weeks after the stereotactic intrathecal injection of tumor cells (using the general method described hereabove), the mice were randomized into 4 treatment groups:
- a group of 13 mice ("CONTROL group") which received no treatment;
- a group of 12 mice ("TMZ group") which received one oral administration of temozolomide per day at 7.5 mg/kg for a week, then no treatment for two weeks, then again one oral administration of temozolomide per day at 7.5 mg/kg for a week, then again no treatment for two weeks, and so on;
- a group of 12 mice ("MCTNT group") which received one oral administration of macitentan at 10 mg/kg every day; and
- a group of 13 mice ("TMZ + MCTNT group") which received both temozolomide and macitentan according to the same doses, administration routes and schedules as for the two previous groups.

The mice were monitored daily and euthanized when they developed neurologic symptoms or became moribund. Days of survival were recorded and brain tissues were harvested and processed for histologic examinations. The experiment was terminated on day 100.

### Results:

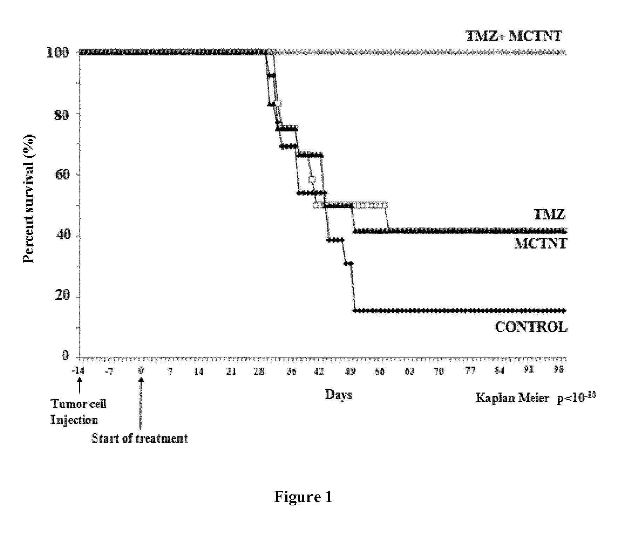

The respective survival results obtained for the four groups of mice are shown in Figure 1. As can be noticed, after 100 days, the survival rate of the CONTROL group was below 20% and the survival of either the TMZ group or the MCTNT group was about 40%; in contrast, the survival of the TMZ + MCTNT group was 100%. Besides, all mice treated with macitentan plus temozolomide were alive at day 100 with no evidence of tumor in the brain.

### Example 2: treatment with macitentan and paclitaxel:

### Experimental_methods:

Two weeks after the stereotactic intrathecal injection of tumor cells (using the general method described hereabove), the mice were randomized into 4 treatment groups:
- a group of 8 mice ("CONTROL group") which received no treatment;
- a group of 8 mice ("PTL group") which received one intraperitoneal administration of paclitaxel per week at 8.5 mg/kg;
- a group of 8 mice ("MCTNT group") which received one oral administration of macitentan at 10 mg/kg every day; and
- a group of 8 mice ("PTL + MCTNT group") which received both paclitaxel and macitentan according to the same doses, administration routes and schedules as for the two previous groups.

The treatment was continued for 70 days, after which the experiment was terminated. Moribund mice were euthanized.

### Results:

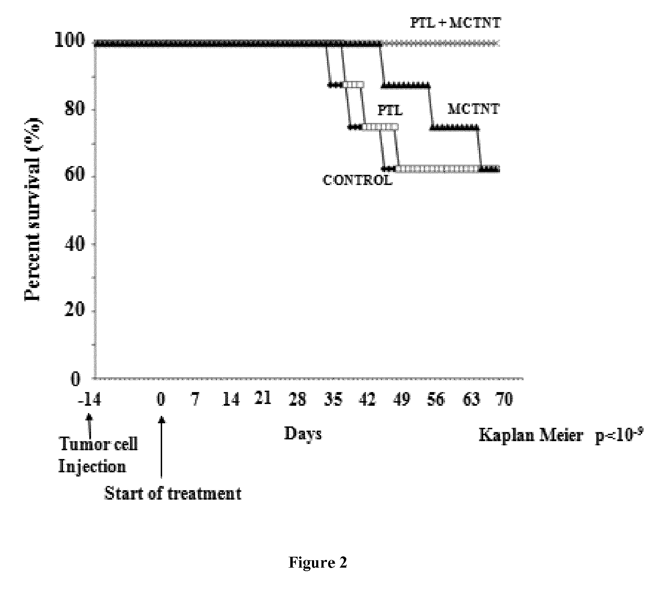

The respective survival results obtained for the four groups of mice are shown in Figure 2. As can be noticed, after 70 days, in the CONTROL group, the PTL group or the MCTNT group about 60% of the animals were still alive; in contrast, at the time of termination, the survival rate of the PTL + MCTNT group was 100% without any signs of disease.

### Example 3: comparison of treatments involving endothelin receptor antagonists in combination with temozolomide:

### Experimental methods:

21 days after the stereotactic intrathecal injection of luciferase labeled LN229 glioblastoma cells (using the general method described here above) (day 0), the mice were randomized into 7 treatment groups:
- a group of 10 mice ("Control group") which received no treatment;
- a group of 10 mice ("TMZ group") which received one oral administration of temozolomide per day at 7.5 mg/kg for a week, then no treatment for two weeks, then again one oral administration of temozolomide per day at 7.5 mg/kg for a week, then again no treatment for two weeks, and so on;
- a group of 10 mice ("Macitentan + TMZ group") which received, on the one hand, one oral administration of macitentan at 10 mg/kg every day and, on the other hand, temozolomide according to the same dose, administration route and schedule as for the TMZ group;
- a group of 10 mice ("Atrasentan group") which received intraperitoneal administration of atrasentan at 10 mg/kg every day;
- a group of 10 mice ("Atrasentan + TMZ group") which received both atrasentan and temozolomide according to the same doses, administration routes and schedules as for the Atrasentan group and the TMZ group respectively;
- a group of 10 mice ("Zibotentan group") which received oral administration of zibotentan at 20 mg/kg every day; and
- a group of 10 mice ("Zibotentan + TMZ group") which received both zibotentan and temozolomide according to the same doses, administration routes and schedules as for the Zibotentan group and the TMZ group respectively.

The treatment was continued for 120 days, after which the experiment was terminated. Moribund mice were euthanized.

### Results:

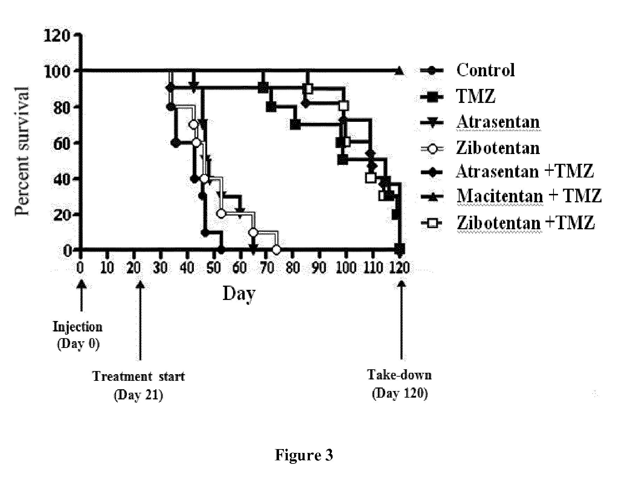

The respective survival results obtained for the seven groups of mice are shown in Figure 3. As can be noticed all mice from the Control group, the Atrasentan group or the Zibotentan group became moribund during the first 50-80 days of the study and had to be euthanized. Animals of the TMZ group, the Atrasentan + TMZ group or the Zibotentan + TMZ group survived longer but at termination of the study all animals were moribund and had to be euthanized; in contrast, at termination of the study at 120 days after tumor cell injection, all mice of the Macitentan + TMZ group were alive without any signs of disease. IVIS Bioluminescence imaging, after treating animals with luciferine, confirmed the minimal residual disease in animals treated with the macitentan + TMZ combination.

Immunohistochemistry of tumors from Examples 1 and 2 using antibodies directed against phosphorylated AKT and MAPK demonstrated strong reduction of both proteins in animals treated with macitentan. The reduction of these proteins which indicate highly active survival and proliferation pathways is much less prominent in tumors of animals treated with atrasentan or zibotentan (this example). Suppression of survival and proliferation pathways sensitizes cells to chemotherapy and could be the reason for the high effectiveness of macitentan in combination with chemotherapy.

## Claims

1. A product containing macitentan, or a pharmaceutically acceptable salt of this compound, for use in the treatment of malignant glioma in combination with with radiotherapy or with radiotherapy and a cytotoxic therapy agent.

2. A product according to claim 1, wherein the malignant glioma are glioblastoma multiforme.

3. The product of claim 1 or 2, wherein macitentan or its pharmaceutically acceptable salt is for use in combination with radiotherapy and a cytotoxic therapy agent, said cytotoxic therapy agent being selected from alkylating agents and mitotic inhibitors.

4. The product of claim 3, wherein the cytotoxic therapy agent is an alkylating agent.

5. The product of claim 4, wherein the alkylating agent is temozolomide or a pharmaceutically acceptable salt thereof.

6. The product of claim 3, wherein the cytotoxic therapy agent is a mitotic inhibitor.

7. The product of claim 6, wherein the mitotic inhibitor is paclitaxel or a pharmaceutically acceptable salt thereof.

8. The product of one of claims 1 to 7, wherein macitentan or its pharmaceutically acceptable salt is intended to be administered by oral route.

9. The product of one of claims 1 to 7, wherein macitentan or its pharmaceutically acceptable salt is intended to be administered by intravenous or intraperitoneal route.

10. Macitentan in a medicament for use in combination with radiotherapy in the treatment of malignant glioma.

11. Macitentan in a medicament for use in combination with radiotherapy according to claim 10, wherein the malignant glioma are glioblastoma multiforme.
